# EUROPEAN PATENT APPLICATION

(11) **EP 0 657 149 A1**
(43) Date of publication of application: **14.06.1995**
(21) Application number: 94500182.4
(22) Date of filing: 15.11.1994
(51) Int. Cl.: A61F 5/01

(54) **Dynamic articulation**

(30) Priority: 09.12.1993 ES 9302559
(71) Applicant: Mata Garcia, Gerardo, ES-13001 Ciudad Real (ES); Garcia Segura, Cesareo, E-13440 Argamasilla de Calatrava (Ciudad Real) (ES)
(72) Inventor: Garcia Segura, Cesareo, Ciudad Real (ES)
(74) Representative: Botella Pradillo, Juan

(57) **Abstract**

Articulation for use on knee or hip of femoral ortheses, being formed by two articulated flat-bars, with 90 degrees swing on the axle of articulation. The upper flat-bar (1) has a box (2) containing bolt (12) with a spiral spring (17). The lower flat-bar (8) has a yoke (10), held by a rivet (9). In the yoke is lodged the articulation axle (6) and a pin (11), supporting the bolt (12). The bolt is threaded (15) and there is a nut (16) to adjust the spiral spring force. On the other end of the bolt there is a semispherical washer (19), making end on the perforated wall (7) of the box (2). The perforated wall allows bolt (12) to pass through when articulation swings. The assembly mechanism is covered by two metallic lids (20).

## Description

### INVENTION OBJECT

The present invention refers to an articulation for use in knee or hip, whose purpose is to help walking, and both the seated and standing positions for these patients with one, or partially both lower extremities invalid caused by diseases such as poliomyelitis, myelomeningocele or any other neurologic defect such as brain paralysis and traumatic atrophys. It maintains the upright posture, and withstands hip and knee bending. Also it makes possible the extension of them when walking and raising up from sitting down posture.

### FIELD OF INVENTION

The invention is relevant to the industry involved in production of orthopaedic devices.

Possibly, one of the oldest relics of orthopaedic devices was that discovered by the HEARST espedition to Egypt, where two sets of wooden splints bound to the fractures of 5.000 year old skeletons were found.

HIPOCRATES AND GALENO tried to adjust deviation of the spiral column using ingenious devices; but it was AMBROSIO PARE who, in 1500, was the first to use metallic corsets wich were perforated to make them lighter and cushioned to avoid skin abrasions.

Medical terminology was enriched in 1741 when NICHOLAS ANDRY, Professor of Medicine at the University of Paris, gave the speciality of Orthopaedics its name. He built on all the efforts made from the ancient times, using mechanical aids that could improve some of the functional aspect of the human locomotor system.

Initially Orthopaedics was defined as "the art to preventing and correcting deformities in the body of children", and used as therapy, basically, instruments placed in the area affected whose, purpose was to correct the deformity. They were orthopaedic apparatus or what we call nowadays ortheses and prostheses.

Althogh Orthopaedics is considered as a speciality derived from Surgery, the surgical methods, that is, Orthopaedic surgery, were not used in the treatment of affections of the organs of mobility until nineteenth century by DELPECK, DUPINSTEN, STROMEYER, LITTLE, etc.

The patients treated by the Othopaedic Services, for deficiencies or deformities of the body, specially the locomotor system, come from specialist areas such as Neurology, Neurosurgery, Paediatrics, Rheumatological Surgery or Internal Medicine.

The two World Wars helped greatly to develop the field of Orthopaedics due to the great number of invalids produced. To over come the problem, Governements invested in research into ortheses and prostheses at Universities and Adaptation Centres.

With the introduction of Biomechanic studies, Orthopaedics has seen an spectacular advance. The application of Physics to the locomotor system was made by STEINDLER, INMAN, CHARNLEY and many others, making possible collaboration between engineers and specialized doctors. This collaboration has provided a better knowledge of kinesiology and, consequently, the introduction of new mechanical devices in therapeutics.

Today research into new materials has been included, thereby adding to the progress made in the construction of orthopaedic apparatus.

Within Orthopaedics, orthopaedic technique has been considered for a long time, as a paramedical craft speciality, and scientifically under developed. From those forerunner, small craftsmen until now, very powerful industries have been emerged in Germany, United Kingdom, USA, etc, capable to fulfilling most of the world demand and, thanks to the importance given to research, of solving technical and aesthetic problems that seemed insurmontable a few years ago.

The Orthopaedic Technique make possible the individual adaptation of invented orthopaedic devices to the characteristics of each patient.

An orthopaedic device or ortheses is a mechanical apparatus that is used for treatnent of illness, traumatic injuries, their consequences and congenital malformations in the locomotor system. The easiest concept that encompasses all that purposes is: "External Corrector Device".

The Ortheses are used:
* To avoid painful or counterproductive movements and to allow all these that arn't or to eliminate all mobility.
* To inmobilize temporarily or permanently one or more articulations.
* To replace the loss of muscles, caused by nerve muscular injury.
* To unload an articulation of body weight after injury.
* To correct any deformity or malformation concerning bones and articulations.
* To prevent any future complications caused by desease or injury that may damage the limb.
* To mantain a position obtained by manipulation or sugical procedures.

There are two main kinds of ortheses, passive or postural and active or functional; they can be used for upper and lower members or the spine.

Within the ortheses of the lower member, we can find lower devices, working on ankle, called single or bi-bar, depending on whether one or two flatbars are used and whose joints may be free, fixed, adjustable or with antiquine spring.

The upper devices may act on two or three articulations; (below the knee they work like a lower device). Generally they've got two upright flat-bars made on duralumin, that come down along the leg, one on the internal side, two centimeters below the perineum to the base of the foot, and the other flat-bar coming down on the external side from four centimeters above the major trochanter to the base of the foot as well. The aforementioned flat-bars are linked by half-rings positioned at the back rear of the leg, horizontal to the ground. There may be trhee or four in number, according to length of the leg. The first half-ring is situated on the upper part of muscle. When it is necessary for the body weight to be unloaded from the extremity through the bi-bar, this half-ring is situated inmediatly under the ischiatic tuberosity, with appropiate shape to avoid pressure on the perineal area.

The second half-ring is positioned on the lower half of the thigh.

The third half-ring is positioned on the upper part of the calf, just under the knee articalation.

Sometimes, it is necessary to place a fourth half-ring just at half the distance between the third half-ring and the ankle articulation.

At the front, the rear half-rings of the ortheses are completed with leather fittings which with buckle and strap (or velcro), fix the ortheses to the leg.

At the level of anatomic articulation of the knee and, sometimes of the hip, mechanical articulations are sometimes added. There are several models of them: Free, locked, voluntarily lockable, adjustable, active, etc.

Depending on the materials used and the construction shape, the ortheses may be metallic, thermoformed or mixed. Although they are also made of carbon fibre, lighter and stronger material.

All that models of ortheses have certain shapes, forms and elements in common. The difference between them consists of the material and the types of articulation. The choice of material depend on whether importance is given to the sturdiness, the weight or the aesthetic aspect of the apparatus.

Protheses are mechanical devices which replace totally or parcially the loss of an organ, attempting to restore ist function.

The forces transmission through a bar does not depend on its shape, rather on the relative position of its extremitys, assuming bar rigidity. If we introduce an articulation in the bar with a limit avoiding movement in more than one direction, the articulation will be more or less steady if it is in a straight line from one extreme to the other.

In orthopaedic devices the mechanics are not so simple. The lower extreme is not fixed to the ground but is joined to a first longer extension and a second shorter one which acts as the foot.

If this extension is rigid, then, for the mid point flexion to occur, the lever force which the lenght of the extension represents must be overcome, otherwise this force tends to displace this point in the opposite direction. The longer the extension, the grater the force to be overcome to achieve flexion. If the joint at the lower end is not rigide, but has a limit to its movement, the length of this limit is that which produces the resistence which opposes the extension. As a result, the lesser the movement allowed by the limit in the lower point, the more stability the articulation in the centre will have and, also, the shorter the "foot" extension, the lesser the resistence offered to the flexion.

Before, in paralysis or deficiencies of a lower member, in order to walk, patients were oblied to use sticks or crutches, or they had to press their hand on the front part of the thigh to stabilize the lower extremity. In the case of unsteady hip, the mobility of this was limited by a blocking of the articulation which connected to belt or pelvic basket.

Specialists in orthopaedia fitted a rigid support connected to the upper part of the thigh and supported in differents areas of the leg, leaving all the lower limb rigid thus permitting a more or less difficult step.

Later on, modifications were made and articulations were connected to the knee and hip, according to the case, which permitted the patients to sit and bend the leg, thus overcoming the necessity patients had of keeping the leg rigid when they wanted to sit down. The articulations permitted flexion of the knee and, at the moment of starting to walk, they were generally blocked by a ring system or that known as the swiss lever.

Later come the quadricepital knee articulation with a socket for the placement of a pivot which has a spiral spring inserted with some orifices in the third part, through which two axles pass sustaining two ball bearings which move along a flat bar.

### INVENTION DESCRIPTION

The articulation of the knee and or hip proposed by this invention is a solution to the problems which patients have when obliged to use a long walking apparatus to substitute the lost of a muscle or group of muscles of the locomotor system essential for walking.

To sum up, the proposed articulation is incorporated into the long walking apparatus, at the level of knee or hip.

The articaulation comprises of upper and lower flat-bars joined by an axle. The lower flat-bar is fastened, by a rivet, to a piece in the form of a yoke which has a central axle which allows a turn of 90 degrees with respect to the upper flat-bar. In the yoke shaped piece there is, behind the axle, a pin connected to the two sides of the yoke.

The upper flat-bar has, in its lower part, a box in which there is a mechanism which consists of a bolt which in its lower part is cylindrical with a slot which is coupled to the pin. Following this a bolt with a thread for the nut which regulate the pressure exerted by the spiral spring restrained in rest of the bolt. At the end of the bolt there is a semispherical washer which serves as stop and presses against a perforated wall that makes room for the bolt when it turns 90 degrees. The box is covered by two metal plates connected at one end by two pivots which connect to an another two situated in the internal part of the front and back walls of the box and hold and fastened by a screw at the other end.

The patient using a long-walking device provided with this articulation, either on knee or hip, can flex these easily and is more secure because his lower limb does not fail. The patient, also, has an active extension upon lifting the leg when walking or assistance in changing from a seated position to a standing without having to manipulate the articulation.

Without any doubt, this articulation improves greatily on articulations with a free axle or, as mentioned before, on these which are blocked by rings when walking and have to be unblocked when the patient sits down.

The dynamic hip-knee articulation also improves on the articulations kown as the Swiss Lock, which has to be blocked by means of a semi-circular ring fitted to the same articulation and acts by means af an elastic connected at one end to the ring and at the other to a lower ring to produce a rigid ortheses on walking and must be unblocked by pressing upwards on the ring.

The dynamic hip-knee articulation clearly improves the knee cuadricepital one, because it transfers greater forces to the lower flat-bar, even with smaller springs. Due to its greater force, dynamic hip-knee articulation does much better than cuadricepital one, being safer, lighter, more aesthetic and usefull for the hip. Both articulations also differ because in this one the spiral spring moves not only in one plane, but in two.

### DRAWINGS DESCRIPTION

Figure number 1 shows a plane and elevated view of the upper flat-bar to make to 90 degree swing. It is situated over the articulation and has a box containing the spiral spring.

Figure number 2 shows a plane and elevated view of the other flat-bar. It is situated in the lower part of the articulation, under its axle. It holds up the articulation by means of a yoke.

Figure number 3 shows bolt, nut, washers, spiral spring and semispherical washer.

Figures numbers 4 & 5 show plane and elevated views of the metallic plates whose purpose is to cover the spiral spring, that is lodged in the part shown in figure number 1.

Figure number 6 shows an assembly plan view with the parts shown in drawings numbers 1, 2, 3, 4 and 5, which constitute the hip-knee articulation.

Figure number 7 shows a frontal elevated view of the assembly plan seen in number 6.

### PREFERABLE REALIZATION OF THE INVENTION

From these figures, one can see that envisaged dynamic hip-knee articulation consists of an upper articulated flat-bar (1), fitted with a box in its lower end (2). Inside of which are two pins (3) and a threaded hole for the screw (4), alognside there is another hole (5) for the plate pin (22).

The box is divided by a perforated wall (7) in its upper part to permit the bolt (12) to pass through when articulation flexes. In the lower part of the box there is a hole (6) that allows for the axle of the articulation. The box (2) can be made indepently of the flat-bar (1) and joined to it by one or two rivets.

The articulation is supported by a flat-bar in its lower part, connected to a yoke (10) by a rivet. In the yoke there are two holes, one on each side, behind the central axle (6), to lodge the split pin (11), where the bolt rests in its lower part by means of a slot (14). This bolt is lodged in the box of the upper bar (1) and has a thread (15) on which a nut (16) turns to graduate tension of the heliciodal spring (17), restrained in the bolt (12). The spiral spring is separated by two washers (18) from the nut (16) and the semispherical washer (19), which touches the perforated wall (7) of the upper end of the box (2). The box (2), containing the mechanism, has two lids (20), one on either side. The lids have two pins (21), coupled to the internal pins (3) of the box, with a pin (22) and a hole (23) to make the coupling in the upper part.

The rear part of the yoke (10) is designed to allow an articulation of 90 degrees and the frontal part of it has a stop (24) to avoid exceeding 0 degrees.

To sum up, it can be said that this type of articulation udertakes the movements of a knee or femoral ortheses hip.

As has alraedy been indicated, the patient using an ortheses with this kind of articulation does not need to manipulate it when sitting down, allowing him to extend the limb directly without impediment and without manipulation.

There is no need to further extend this description for any expert in the subject to understand the scope of the invention and all the advantages that go with it.

The materials, shape, size and positioning of the elements will be subjected to variation as long as this does not alter the essential character of the invention.

The terms in which this report has been written is a wide and not limiting sense.

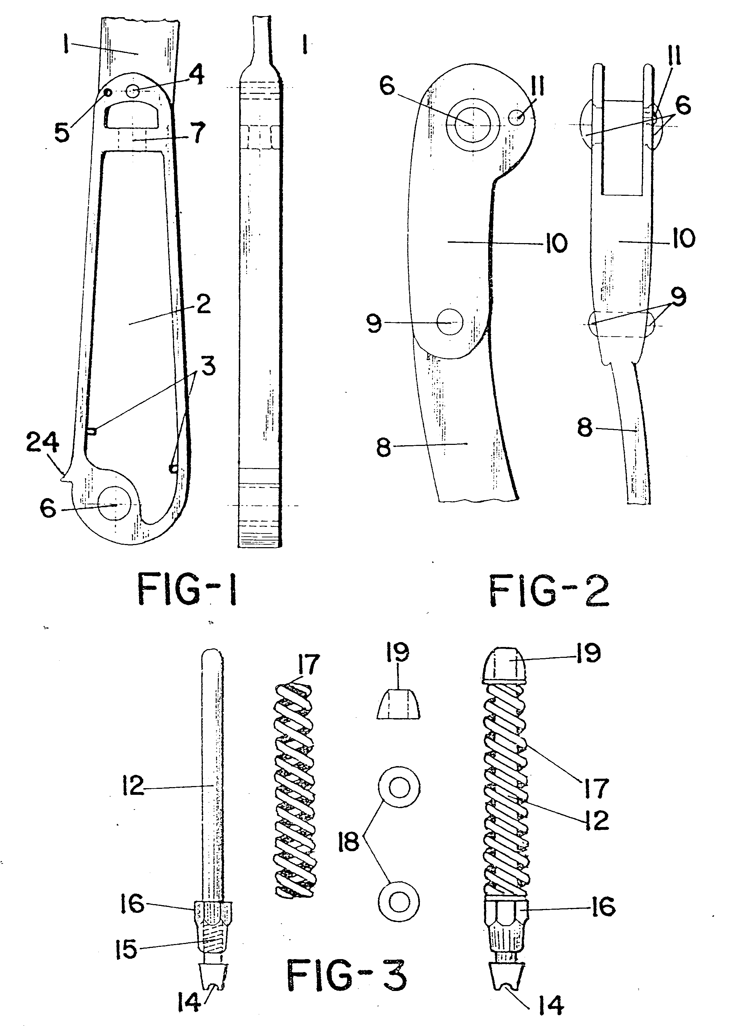

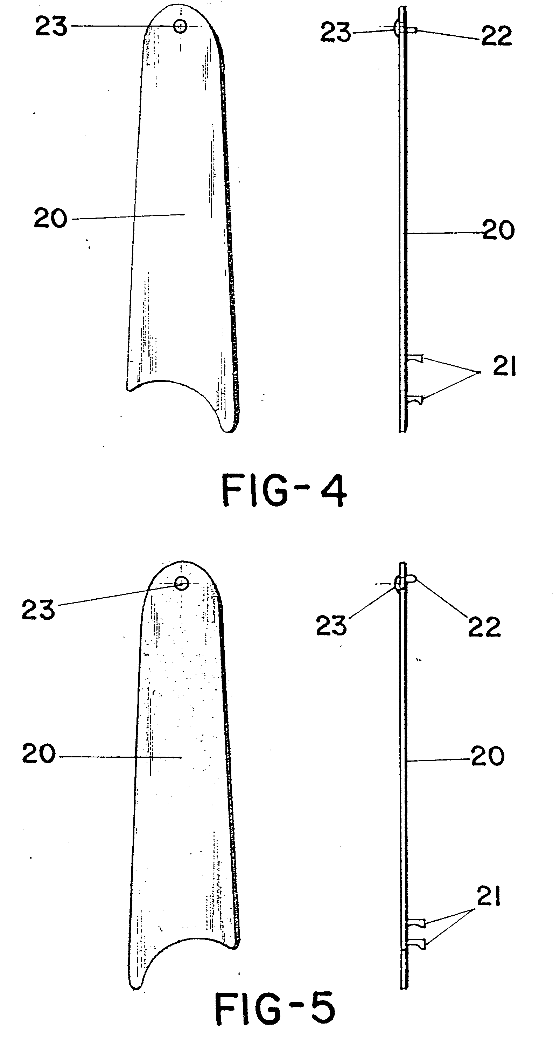

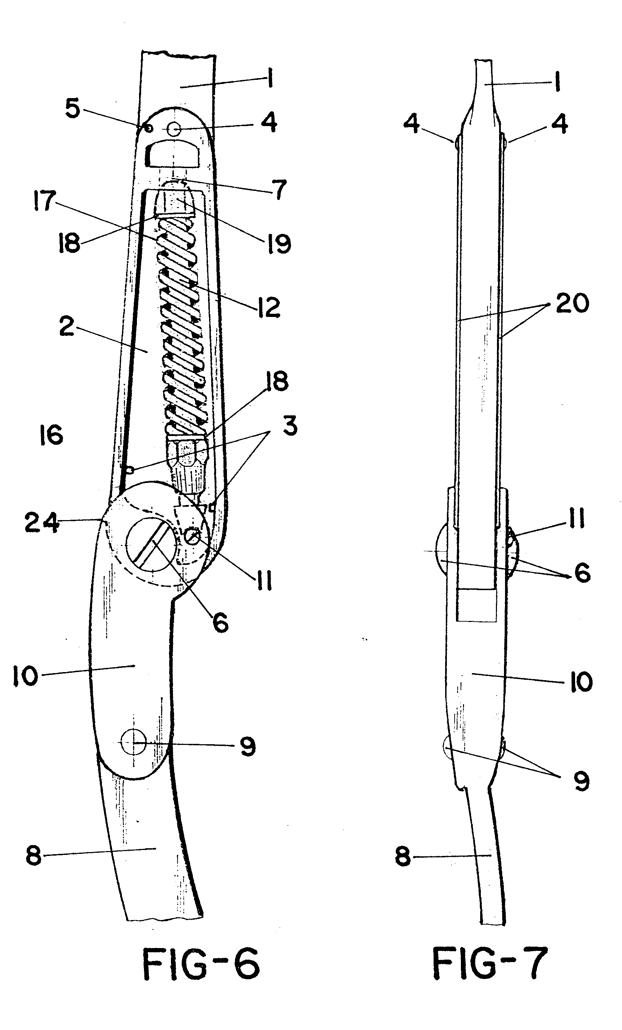

## Claims

1. Dynamic articulations for use on knee or hip of femoral ortheses whose main charasteristic is that is formed by upper (1) and lower (8) flat-bars, joined by an axle (6), located on a yoke (10), restrained by a rivet (9) to the lower fla-bar; behind the axle, a bolt (11) joins both sides of the yoke (10); the lower part of the upper flat-bar (1) has a box (2), where is lodged the bolt (12), with cylindrical-shaped end, with a slot (14) in its distal face, coupled to the pin (11), thread (15) on the middle third portion, with hexagonal nut (16), and an extension on its upper end where the spiral spring (17) is restrained in contact with the semispherical washer (19) and that presses against a perforated wall (7) and a lid for internal and external coverage (20).

2. Dynamic articulation, according to first claim, is essentially characterized by a yoke shaped part (10) situated on the upper part of the lower flat-bar.

3. Dynamic articulation, according to former claims, is essentially characterized because behind the swing axle (6), the yoke (10) sides are joined by a bolt (11).

4. Dynamic articulation, according to former claims, is essentially characterized by a bolt (12), positioned in the box of the upper flat-bar, with its lower end (14) with cylindrical shape and slot finished, followed by thread (15) and extension where an spiral spring (17) is restrained.

5. Dynamic articulation, according to former claims, is essentially characterized because the upper end of the spiral spring (17) is stopped with a semispherical washer (19).

6. Dynamic articulation, according to former claims, is essentially characterized by a nut (16) threaded on a bolt (12), to adjust spiral spring (17) pressure.

7. Dynamic articulation, according to former claims, ia essentially characterized because the box has a perforated wall (7), at the upper end, where the semispherical washer (19) is positioned to allow the bolt (12) to pass when turning.

8. Dynamic articulation, according to former claims, is essentially characterized because the frontal wall of the box (2) has a stop (24) for the extended articulation.

9. Dynamic articulation, according to former claims, is essentially characterized because the back wall of the yoke is designed to bend the articulation to a maximum angle of 90 degrees.

10. Dynamic articulation, according to former claims, is essentially characterized because the lower end (14) of the bolt (12) , held up by pin (11), moves with this when the turn is made.

11. Dynamic articulation, according to former claims, is essentially characterized by the fact that the upper, flat-bar has two unthreaded holes, one at each side, and two threaded holes, one at each side, all of them situated in the upper part of the box, with two internal pins (3), on the lower end of the box, holding up the outer and inner lids by bolts (21), screws, and plates.
